**Europäisches Patentamt**

**European Patent Office**

**Office européen des brevets**

(19)

(11) Veröffentlichungsnummer: **0 210 340 B1**

# EUROPÄISCHE PATENTSCHRIFT

(12)

(45) Veröffentlichungstag der Patentschrift: 17.07.91

(21) Anmeldenummer: 86105171.2

(22) Anmeldetag: 15.04.86

(51) Int. Cl.5: **A61B 5/042**, A61M 25/01, A61B 5/06

(54) Vorrichtung zur Lagekontrolle eines zentralvenösen Katheters.

(30) Priorität: 26.07.85 DE 3526738

(43) Veröffentlichungstag der Anmeldung:
04.02.87 Patentblatt 87/06

(45) Bekanntmachung des Hinweises auf die
Patenterteilung:
17.07.91 Patentblatt 91/29

(84) Benannte Vertragsstaaten:
AT BE CH DE FR GB IT LI LU NL SE

(56) Entgegenhaltungen:
EP-A- 0 082 504
DE-A- 2 736 623
DE-A- 3 100 547
DE-A- 3 120 012
DE-U- 8 509 649

(73) Patentinhaber: **B. Braun-SSC AG**
**Gerliswilstrasse 74**
**CH-6020 Emmenbrücke(CH)**

(72) Erfinder: **Rycyk, Manfred, Dipl.-Ing.**
**Herkulesstrasse 19**
**W-3501 Körle(DE)**
Erfinder: **Schmidt, Klaus**
**Brandenburgerstrasse 16**
**W-3501 Ahnatal(DE)**

(74) Vertreter: **von Kreisler, Alek, Dipl.-Chem. et al**
**Deichmannhaus am Hauptbahnhof**
**W-5000 Köln 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf eine Vorrichtung zur Lagekontrolle eines zentralvenösen Katheters gemäß den Oberbegriffen der Patentansprüche 1 und 2. (DE-U-8509649)

Beim Verlegen eines zentralvenösen Katheters ist es wichtig, die Lage der Spitze des Katheters zu kennen, bevor z.B. Medikamente oder Lösungen zugeführt werden bzw. Blut für entsprechende Analysezwecke entnommen wird. Eine solche Lagekontrolle ist beispielsweise durch Beobachtung der Katheterbewegung unter dem Röntgenschirm möglich. Eine Röntgenüberwachung ist jedoch nicht immer unmittelbar nach der Punktion des Blutgefässes durchführbar und außerdem bedeutet dieses Vorgehen eine Belastung für den Patienten.

Eine andere Möglichkeit der Katheter-Lagekontrolle macht sich EKG-Kurven zunutze, die die Position der Katheterspitze erkennen lassen. Bei einer bekannten Vorrichtung für diesen Zweck (DE-U 82 29 899) ist ein Zwischenstück mit männlichem und weiblichem Konus und durchgehendem Flüssigkeitskanal vorgesehen, in dem ein elektrisch leitender Draht freiliegt. Der eine Konus des Zwischenstückes ist an den Katheteransatz angeschlossen, während mit dem anderen Konus ein von einem Infusionsbehälter herkommender Infusionsschlauch verbunden ist. Zur Lagekontrolle der Spitze des Katheters beim Eintritt in das Herz wird der Katheter mit Elektrolytflüssigkeit aufgefüllt und es werden dann die durch die Elektrolytflüssigkeit geleiteten elektrischen Herzimpulse über den elektrisch leitenden Draht in dem Zwischenstück zu dem EKG-Gerät geleitet. Bei dieser bekannten Vorrichtung ist nachteilig, daß zur Anbringung des Zwischenstückes immer die Verbindung zwischen Infusionsschlauch und Katheter gelöst werden muß, was umständlich ist und auch Sterilitätsprobleme mit sich bringen kann.

Zur Überwindung dieser Nachteile ist in einer anderen bekannten Vorrichtung (DE-U 83 24 566) die Flüssigkeits-Verbindungsleitung zwischen Katheter und Infusionsbehälter mit Hilfe eines in dem Zwischenstück vorgesehenen Dreiwegehahns unterbrechbar ausgebildet und es ist ein freiliegendes elektrisches Kontaktelement eines Verschlußstopfens oder einer elektrisch leitenden Spritze an einen der Wege des Dreiwegehahns angeschlossen. Zwar kann hierbei durch Verstellung des Dreiwegehahns die elektrolytische Flüssigkeitsverbindung zwischen dem elektrischen Kontaktelement und dem Katheter nach Bedarf von der Flüssigkeitsverbindungsleitung zwischen Katheter und Infusionsbehälter getrennt werden, wodurch die Handhabung gegenüber der anderen bekannten Vorrichtung erleichtert wird. Jedoch bleibt der Einbau des Zwischenstückes zwischen Katheter und Infusionsschlauch sowie der Anschluß von Verschlußstopfen oder Spritze umständlich, weil mehrere Konusanschlüsse zusammengesteckt werden müssen. Eine sterile Handhabung der Teile z.B. innerhalb einer Schutzhülle, ist nicht ohne weiters möglich. Auch bleibt die Füllung des Leitungssystems mit Elektrolytflüssigkeit, z.B. Kochsalzlösung, notwendig, die zeitaufwendig und umständlich ist, weil sie langsam und sorgfältig durchgeführt werden muß, damit keine Luftblasen in das Leitungssystem einschließlich eines "intrakardialen EKG's" auf dem Monitor verhindern. Wenn die Infusionsflüssigkeit keine elektrolytische Flüssigkeit ist, kann die EKG-Ableitung erst nach der Katheterverlegung erfolgen.

Weiterhin kann beim Einführen des Katheters zur Versteifung des relativ elastischen Katheterschlauches kein Mandrin zur Erhöhung der Führungsstabilität eingesetzt werden, da das Lumen mit der elektrisch leitenden Flüssigkeit gefüllt werden muß. Dies führt bei den heute verwendeten elastischen und weichen Kathetermaterialien zu Einführungsschwierigkeiten bei der Plazierung des Katheters.

DE-U-85 09 649 beschreibt eine Vorrichtung zur Lagekontrolle eines zentralvenösen Katheters durch EKG-Ableitung der in den Oberbegriffen der Patentansprüche 1 und 2 umrissenen Art. Um bei einer solchen Vorrichtung Perforationen der Gefäßwand mit der metallischen Spitze des Metallstranges zu verhindern, ist die Kunststoffummantelung des Metallstranges über seine Spitze und über die Spitze des Katheters hinaus verlängert. Die Verlängerung ist halbkreisförmig gekrümmt und ihr Ende ist verschlossen. Da zur Übertragung der zu messenden Aktionspotentiale "blanke" Metallstellen mit Blut in Berührung kommen müssen, ist die Kunststoffummantelung des Metallstranges im Bereich seines patientennahen Endes mit mindestens einer Aussparung versehen, die sich innerhalb des Lumens des Katheters befindet. Die Elektrodenfenster sind also durch den Katheterschlauch abgedeckt und nur das in den schmalen Zwischenraum zwischen Führungsmandrin und Innenseite des Katheterschlauches eingedrungene Blut steht als Übertragungsmedium für die bioelektrischen Signale zur Verfügung. Die Vermeidung der Perforation der Gefäßwand wird in diesem Falle mit einer sehr kleinen Kontaktfläche des Metallstranges erkauft, deren Freihaltung zudem nicht gewährleistet ist. Der weiche Katheterschlauch umgibt den Elektrodenfensterbereich mit geringem Abstand und es ist durchaus möglich, daß beim Vorschub der Vorrichtung beim Passieren von Gefäßverengungen der Katheter gegen die Außenfläche des Mandrins gedrückt wird und Elektrodenfenster ganz oder teilweise verschließt. Ferner kann das Erscheinen eines intrakardialen EGKs auf dem Monitor durch Luftblasen verhindert werden, die in den Ringraum zwischen Katheter und Mandrin eindringen und das oder die Elektrodenfenster zusetzen. Der bei der bekannten Vorrichtung wichtige Abstand zwischen dem Mandrin und dem Katheter ist im übrigen nachteilig, weil er zuläßt, daß Blut durch den Ringspalt nach hinten strömt und sofort austritt, wenn mit dem Herausziehen des Mandrins aus dem Katheter begonnen wird. Dies ist eine gefährliche Infektionsquelle für den Anwender.

Bei einem Führungsstab gemäß DE-A-31 20 012 liegt die Spitze des Metallstranges an der Spitze des Katheters, wodurch zwar eine größere Kontaktfläche vorhanden ist, jedoch die Gefahr besteht, daß die metallische Spitze des Führungsstabes beim Vorschieben des Katheters die Gefäßwand perforiert. Auch lebensbedrohendes Herzflimmern kann eintreten, wenn die Metallspitze die Herzwand berührt und störende Potentiale auf die Herzwand übertragen werden.

Der Erfindung liegt die Aufgabe zugrunde, die bekannte patientenschonende Vorrichtung zur Lagekontrolle eines zentralvenösen Katheters nach DE-U-85 09 649 so zu verbessern, daß die Ableitung eines einwandfreien EKGs sichergestellt ist.

Diese Aufgabe wird erfindungsgemäß durch zwei parallele Ausprägungen gelöst, die in den kennzeichnenden Teilen der Patentansprüche 1 und 2 definiert sind.

Im einen Fall steht die gesamte Querschnittsfläche und im anderen Fall die über eine bestimmte Länge abisolierte Spitze des Metallstranges als Kontaktfläche zur Verfügung, die zuverlässig freigehalten ist und großflächige Berührung mit Blut zur Übertragung kräftiger Signale ermöglicht. Der Mandrin kann das Lumen des Katheters spaltfrei ausfüllen, so daß Blutrückstrom durch einen Ringraum zwischen Mandrin und Katheter verhindert wird. Da die blanke Spitze des Metallstranges mit Abstand hinter der axialen Öffnung der Spitze des Katheters zurückgezogen ist, verhindert die weiche Katheterspitze Perforationen der Gefäßwand durch den Metallstrang und elektrostatische Aufladungen mit schädlichen Einflüssen auf den Herzrhythmus können nicht an die Herzwand gelangen.

Die elektrischen Herzimpulse werden durch den elektrische leitfähigen Mandrin direkt von der Spitze des Katheters zu einem an den Kontaktteil des Mandrins angeschlossenen EKG-Gerät geleitet und die EKG-Kurven zeigen auf dem Monitor die Lage der Katheterspitze an. Die Ableitung über den elektrischleitenden Mandrin erfolgt über Klebeelektroden. Wenn die EKG-Kurve die korrekte Verlegung des zentralvenösen Katheters anzeigt, wird der Mandrin aus dem Katheter herausgezogen und es kann an den Katheteransatz in üblicher Weise ein Infusionsschlauch o.dgl. angeschlossen werden. Dies ist eine wesentliche Erleichterung für den Anwender, der bisher nach der Verlegung des Katheters mit Hilfe eines Mandrins diesen aus dem Katheter herausziehen mußte und dann erst die Kontrollvorrichtung anschließen, elektrolytische Flüssigkeit einfüllen, und die Lagekontrolle durchführen konnte. Eine nachträgliche Lagekontrolle verlangt die Wiedereinführung des Mandrins nach Abkopplung der Kontrollvorrichtung. Die Sicherheit der Katheterverlegung für den Patienten wird durch die erfindungsgemäße Vorrichtung dadurch beträchtlich erhöht, daß die Verlegung und die Lagekontrolle im gleichen Arbeitsgang erfolgen und kürzere Zeit für die korrekte Verlegung benötigt wird. Außerdem ist es möglich, sowohl die Katheterverlegung als auch die EKG-Ableitung unter erhöhten Sterilitätskautelen durchzuführen, weil der Katheter einschließlich Mandrin über seine ganze Länge in einer Schutzhülle untergebracht und gehandhabt werden kann.

Eine vorteilhafte Ausgestaltung der Erfindung ist in Patentansprüche 5 gekennzeichnet. Die Kupplung von Mandrinansatz und Katheteransatz verbindet Katheter und Mandrin zu einer gemeinsam in der Vene vorschiebbaren Einheit, die über den Kontaktteil an das EKG-Gerät angeschlossen ist. Bei miteinander verbundenen Katheter- und Mandrinansätzen ist die Spitze des Mandrins mit Abstand hinter der axialen Öffnung des Katheters zurückgezogen. Hierdurch wird die Einführung der Katheterspitze in das Herz erleichtert, weil die von dem Mandrin freie Spitze des Katheters ausreichend weich und flexibel ist. Außerdem ermöglicht der mandrinfreie Spitzenbereich des Katheters die Anbringung von mit dem freien Lumen des Katheters in Verbindung stehenden Wandlochungen, die verhindern, daß während der Einführphase des Katheters Luftblasen in dem Katheter aufsteigen, die zu Verfälschungen des EKG's führen. Es sind vorteilhafterweise direkt oberhalb (jenseits) der Mandrinspitze mehrere kleine seitliche Lochungen umfangsmäßig verteilt in der Wand des Katheters angebracht, durch die Luftblasen entweichen, bevor sie zu der Spitze des elektrisch leitfähigen Mandrins gelangen und die EKG-Anzeige zur Lagekontrolle beeinträchtigen können. Diese Lochungen sind so klein, daß sie keine Schwächung der Knickstabilität des Katheters zur Folge haben, jedoch groß genug sind, um die Luftblasen austreten zu lassen.

Der Metallstrang kann als Litze aus verseilten rostfreien Stahldrähten gebildet sein. Durch diese Gestaltung des Metallstranges in der Kunststoffummantelung erhält der Mandrin gute elektrische Leitfähigkeit verbunden mit ausreichender flexibler Steifigkeit zur Versteifung des dünnwandigen Katheters.

In der Zeichnung sind Ausführungsbeispiele der Erfindung schematisch dargestellt.

Es zeigen:

Fig. 1     eine perspektivische Gesamtansicht der Vorrichtung zur Lagekontrolle eines zentralvenösen Katheters,

Fig. 2     das patientenferne Ende der Vorrichtung in Draufsicht und vergrößertem Maßstab,

Fig. 3     einen Längsschnitt durch das patientenferne Ende des Mandrins mit Mandrinansatz,

Fig. 4     eine Stirnansicht des Mandrinansatzes in Richtung des Pfeiles IV in Fig. 3,

Fig. 5     einen Längsschnitt durch eine Ausführungsform der Spitzen von Katheter und Mandrin und

Fign. 6     bis 9 verschiedene Ausbildungen der Spitzen von Katheter und Mandrin.

Zu der Vorrichtung zur Lagekontrolle eines zentralvenösen Katheters gehört eine in der Zeichnung nicht dargestellte Punktionskanüle aus Stahl, die von einer Einführhülse 1 koaxial umgeben ist. Die Einführhülse 1 ist dünnwandig ausgebildet und an ihrer leicht angeschäfteten Vorderspitze 2 mit einem Längsschlitz 2 versehen, der eine spätere Aufweitung dieses Bereiches ermöglicht. Das hintere Ende der Einführhülse 1 ist mit einem Ansatzstück 4 verbunden, in dem ein Innenkonus ausgebildet ist und das eine radial nach außen gerichtete Griffplatte 5 aufweist.

Die Einführhülse 1 wird gemeinsam mit der Punktionskanüle in ein Blutgefäß eingeführt, woraufhin die Punktionskanüle aus der Einführhülse 1 axial herausgezogen wird und das Ansatzstück 4 zum Anschluß einer Vorrichtung 10 zur Lagekontrolle eines zentralvenösen Katheters 11 zur Verfügung steht.

Die Vorrichtung 10 umfaßt den kontrolliert zu verlegenden langen schlauchförmigen Katheter 11, mit dessen patientenfernem Ende ein rohrförmiger Katheteransatz 12 mit einem Außenkonus 12a befestigt ist, auf dem ein den Außenkonus 12a umgebendes ebenfalls rohrförmiges Kupplungsstück 13 drehbar angeordnet ist. Das Kupplungsstück 13 ist auf seiner Innenfläche mit einem Innengewinde ausgestattet, das eine Verriegelung mit radialen Vorsprüngen 4a auf der Außenfläche des Ansatzstückes 4 der Einführhülse 1 ermöglicht, wenn der Außenkonus 12a in den Innenkonus des Ansatzstückes 4 eingesteckt ist. Der Katheter 11 ist von einer Schutzhülle 14 aus durchsichtiger dünner Kunststoffolie umgeben, die bei 15 durch Verschweißung verschlossen und bei 16 an einem rohrförmigen Anschlußstück 17 befestigt ist. Die Schutzhülle hält den Katheter 11 und die mit ihm verbundenen Teile steril. Das rohrförmige Anschlußstück 17 ist auf das rückwärtige Ende eines Einführstückes 18 klemmend, aber axial abziehbar aufgesteckt. Das Einführstück 18 besteht aus zwei Längshälften, die nach Abnahme des Anschlußstückes 17 mit Hilfe von quergerichteten Laschen 19 zur Öffnung des Einführstückes 18 auseinandergedrückt werden können. Das Einführstück 18 ist an seinem vorderen Ende mit einem Außenkonus 20 versehen, der in den Innenkonus des Ansatzstückes 4 einsteckbar ist, um die Einführhülse 1 mit dem Einführstück 18 zu kuppeln.

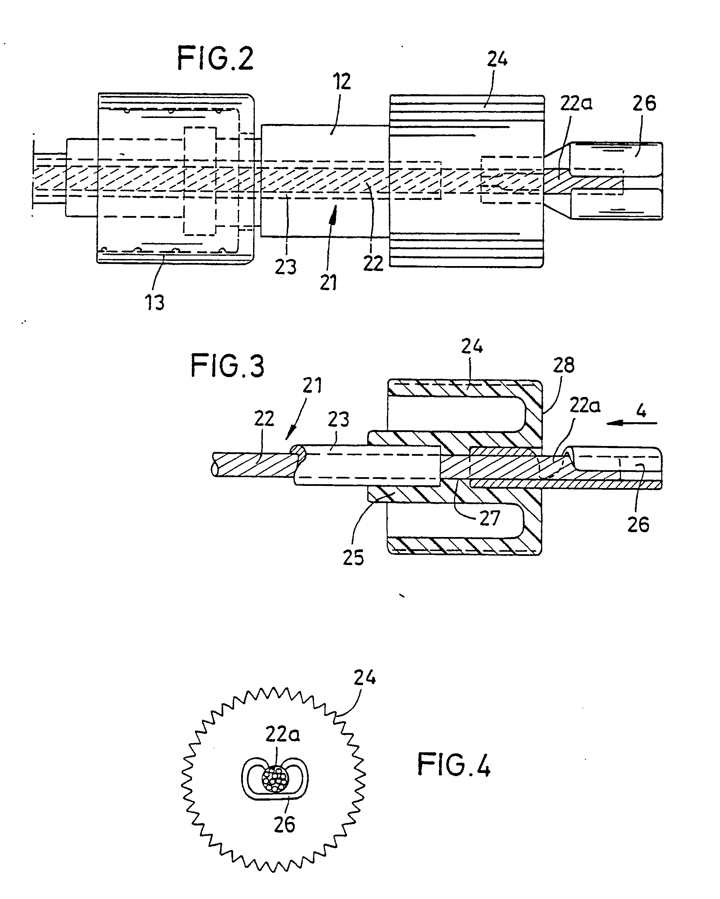

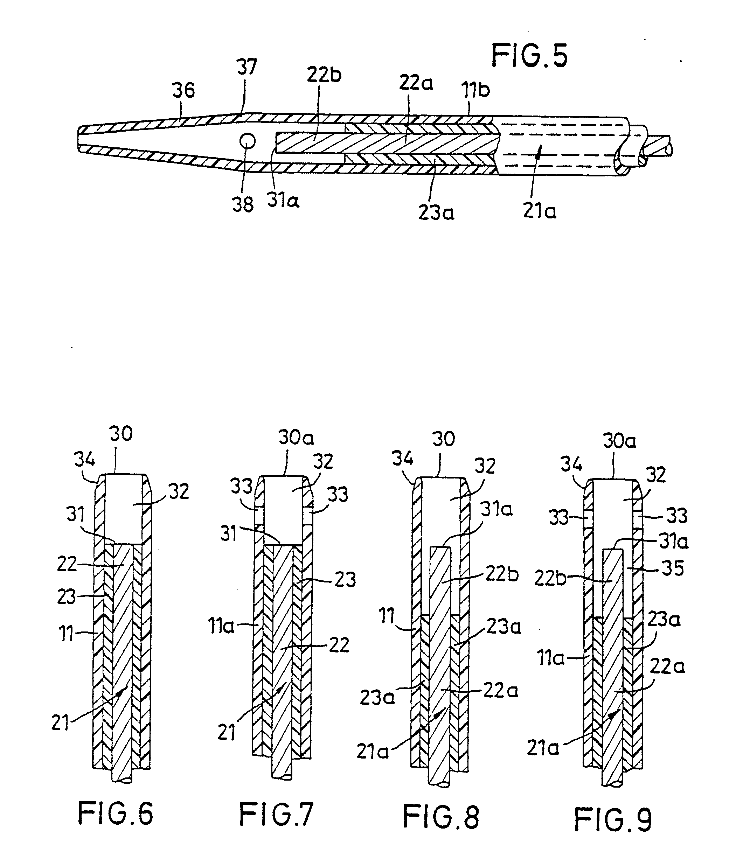

Das Lumen des Katheters 11 wird von einem Mandrin 21 ausgefüllt, der aus einer inneren Litze 22 aus verseilten rostfreien Stahldrähten mit einem Mantel 23 aus Kunststoff gebildet ist (Fig. 3). Mit dem patientenfernen Ende des Mandrins 21 ist ein kappenförmiger Mandrinansatz 24 verbunden. Der Mandrinansatz 24 enthält innen einen zentralen Außenkonus 25 der über den Rand des Mandrinansatzes 24 etwas vorsteht und der von einem an beiden Enden offenen axialen Kanal 27 durchbohrt ist, durch den der Mandrin 21 so hindurchgesteckt ist, daß sein hinteres abisoliertes Ende 22a nach Durchqueren eines Teiles des Kanals 27 ein Stück über die Abschlußwand 28 des Mandrinansatzes 24 übersteht. In dem äußeren Abschnitt des Kanals 27 des Mandrinansatzes 24 ist ein Ende eines Kontaktschuhs 26 aus Metall befestigt, der auf das von der Kunststoffumantelung 23 befreite Ende 22a der Litze 22 aufgeklemmt ist. Der Kontaktschuh 26 ist außerhalb des Mandrinansatzes 24 im Querschnitt B-förmig gebogen und erlaubt den lösbaren Aufsteck-Anschluß eines passenden Kontaktstückes eines zu einem nicht gezeichneten EKG-Gerät führenden elektrischen Leiters. Der Mandrin 21, dessen Kunststoffummantelung 23 in den vorderen Abschnitt des Kanals 27 des Mandrinansatzes 24 hineinreicht, erstreckt sich durch den gesamten Katheter 11 bis zu dessen Spitze, mit der er jedoch nicht bündig abschließt, wie die Fign. 5 bis 9 erkennen lassen. Der Außenkonus 25 dient der Einsteckverbindung mit dem Katheteransatz 12 zur einheitlichen Handhabung von Katheter 11 und Mandrin 21.

Gemäß Fig. 6 und 7 sind die Katheter 11, 11a und der Mandrin 21 mit von einem Ende zum anderen gleichbleibendem Innen- bzw. Außendurchmesser versehen, und der Mandrin 21 füllt das Lumen der Katheter 11, 11a im wesentlichen spaltfrei aus. Hinter der axialen Öffnung 30, 30a jedes Katheters 11, 11a endet der Mandrin 21 mit gerade abgeschnittener stumpfer Spitze, bei der die kreisförmige Stirnfläche 31 der Litze 22 als elektrisch leitende Fläche wirksam ist, über die die EKG-Kurve abgeleitet wird, die anzeigt, wenn die Katheter 11, 11a mit Mandrin 21 in die richtige Position im Herzen eingeführt worden ist. Der von dem Mandrin 21 freie Spitzenteil 32 jedes Katheters 11, 11a ist infolge seiner verstärkungslosigkeit ausreichend nachgiebig, um schonend vorgeschoben werden zu können. Zweckmäßigerweise ist die Außenkante 34 der Katheter 11, 11a an dem Rand ihrer Öffnung 30, 30a von vorne nach hinten schräg auswärts verlaufend abgeschrägt, so daß die Weichheit des Katheters 11, 11a in dieser für die Einführung kritischen Zone gesteigert wird. Damit während der Einführphase keine Luftblasen in den Katheter eintreten, die zu einer Verfälschung des EKG's führen können, sind direkt oberhalb des stumpfen Endes des Mandrins 21 in der Wand des Katheters 11a seitliche Lochungen 33 angebracht, durch die die Luftblasen entweichen. Die Lochungen 33 sind sehr klein und mit ausreichendem Abstand über den Umfang des Katheters 11a verteilt.

In Verbindung mit den Kathetern 11 und 11a wird gemäß Fign. 8 und 9 ein abgewandelter Mandrin 21a benutzt. Dieser unterscheidet sich von dem Mandrin 21 dadurch, daß der Kunststoffmantel 23a an der Spitze des Mandrins 21a über eine bestimmte Länge abisoliert ist, so daß die Litze 22a mit einem Abschnitt 22b über die Stirnkante des Kunststoffmantels 23a vorsteht, wodurch zwischen der Innenwand jedes Katheters 11, 11a und dem Außenumfang des Litzenabschnittes 22b ein Ringraum 35 entsteht und die leitende Fläche der Litze 22a vergrößert wird, weil nicht nur die kreisförmige Stirnfläche 31a der Litze 22a, sondern auch ein Teil ihrer Umfangsfläche als Kontaktelement zur Verfügung steht. Die Stirnfläche 31a liegt mit Abstand hinter der Öffnung 30, 30a jedes Katheters 11, 11a. Bei Vorhandensein von Lochungen 33 (Fig. 9) befinden sich die Lochungen ein kleines Stück vor der Stirnfläche 31a der Litze 22a.

Bei dem Beispiel der Fig. 5 ist der Mandrin 21a in einem Katheter 11b untergebracht, der etwa bis zur Stirnfläche 31a der Litze 22a innen und außen zylindrisch ausgebildet ist und dessen Spitze 36 sich dann nach vorne verjüngt. An dem Übergang 37 zwischen zylindrischem und konischem Teil des Katheters 11b befinden sich mit Abstand zu der Stirnfläche 31a der Litze 22a Lochungen 38 zum Auslaß von Luftblasen.

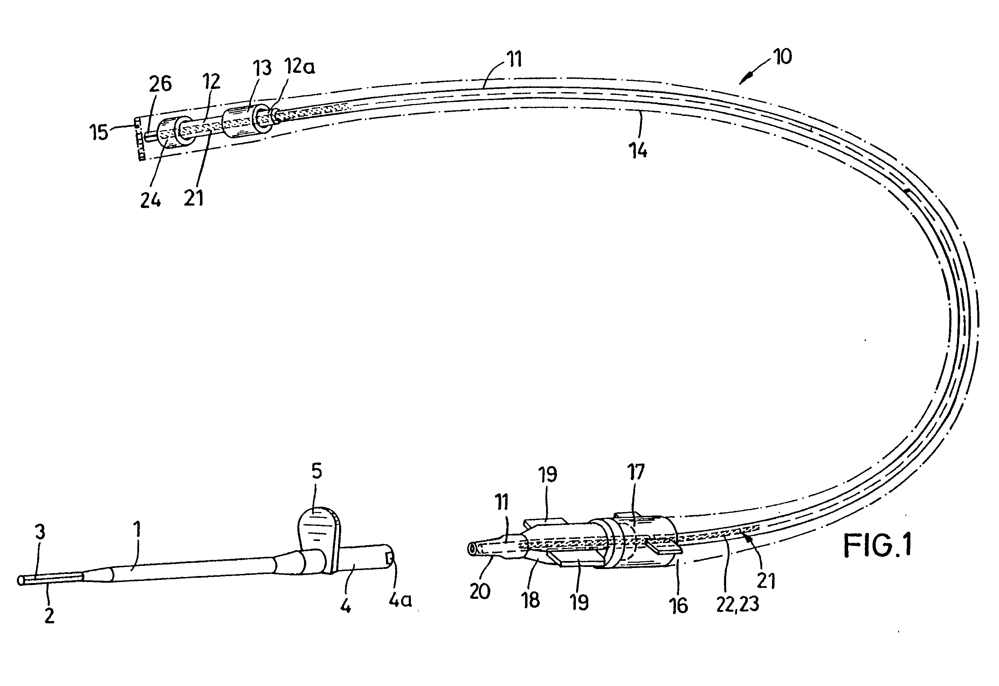

Die in den Fig. 5 bis 9 gezeigten Abstände zwischen der axialen Öffnung jedes Katheters 11, 11a, 11b und der elektrisch leitenden Stirnfläche 31, 31a jedes Mandrins 21, 21a ergeben sich, sobald der Mandrinansatz 24 mit dem Katheteransatz 12 zusammengesteckt ist (Fign. 1 und 2), so daß beim Vorschieben des Katheters mit Mandrin die gewünschte Spitzenanordnung nach Fig. 5 bis 9 ohne Zutun des Anwenders beibehalten wird. Bei der steril verpackten Anordnung gemäß Fig. 1 ragt die Spitze des Katheters 11, 11a, 11b in den Außenkonus 20 des Einführstückes 18 hinein und wird aus diesem unmittelbar in den Kanal der nach Herausziehen der Punktionskanüle mit dem Außenkonus 20 zusammengesteckten Einführhülse 1 hineingeschoben, die in dem Blutgefäß liegt.

Nach Verlegung des Katheters 11, 11a, 11b und Erkennung der die richtige Lage anzeigenden EKG-Kurve auf dem Monitor wird der Mandrin 21, 21a aus dem Katheter 11, 11a, 11b herausgezogen. Sodann wird das Anschlußstück 17 von dem Einführstück 18 getrennt und das Einführstück 18 durch Längsteilung von dem Katheter abgenommen, so daß der Außenkonus 12a des Katheteransatzes 12 in den Innenkonus des Ansatzstückes 4 der Einführhülse 1 eingeführt und das Kupplungsstück 13 auf den radialen Vorsprüngen 4a verriegelt werden kann. An den Katheteransatz 12 kann dann eine Spritze, ein Infusionsschlauch o.dgl. angesetzt werden.

**Patentansprüche**

1. Vorrichtung zur Lagekontrolle eines zentralvenösen Katheters durch EKG-Ableitung, bestehend aus einem schlauchförmigen Katheter (11;11a) mit Katheteransatz (12) und aus einem in das Lumen des Katheters (11;11a) eingefügten Mandrin (21), der aus einem kunststoffummantelten Metallstrang gebildet ist, wobei die Spitze des Metallstranges mit Abstand hinter der axialen Öffnung (30;30a) der Spitze des Katheters (11;11a) zurückezogen ist und das patientenferne Ende des Metallstranges einen elektrischen Kontaktteil (26) zum Anschluß an ein EKG-Gerät trägt,
   **dadurch gekennzeichnet,**
   daß die Spitze des Metallstranges des Mandrins (21) mit dem Rand seines Kunststoffmantels (23) bündig endet.

2. Vorrichtung zur Lagekontrolle eines zentralvenösen Katheters durch EKG-Ableitung, bestehend aus einem schlauchförmigen Katheter (11;11a;11b) mit Katheteransatz (12) und aus einem in das Lumen des Katheters (11;11a;11b) eingefügten Mandrin (21a), der aus einem kunststoffummantelten Metallstrang gebildet ist, wobei die Spitze des Metallstranges mit Abstand hinter der axialen Öffnung (30;30a) der Spitze des Katheters (11;11a;11b) zurückgezogen ist und das patientenferne Ende des Metallstranges einen elektrischen Kontaktteil (26) zum Anschluß an ein EKG-Gerät trägt, dadurch gekennzeichnet, daß die Spitze des Metallstranges des Mandrins (21a) mit einem Abschnitt (22b) über den Rand seines Kunststoffmantels (23a) vorsteht.

3. Vorrichtung nach Anspruch 1 oder 2, dadurch gekennzeichnet, daß die Wand des Katheters (11a;11b) in dem mandrinfreien Bereich seiner Spitze (32;36) gelocht ist.

**4.** Vorrichtung nach Anspruch 3, dadurch gekennzeichnet, daß mehrere kleine Lochungen (33;38) umfangsmäßig verteilt angeordnet sind.

**5.** Vorrichtung nach Anspruch 1 oder 2, dadurch gekennzeichnet, daß das patientenferne Ende des Metallstranges über das Ende des Katheters (11;11a;11b) vorsteht und mit einem kappenförmigen Mandrinansatz (24) fest verbunden ist, der mit dem Katheteransatz (12) lösbar kuppelbar ist und der den Kontaktteil (26) trägt.

**6.** Vorrichtung nach einem der Ansprüche 1-5, dadurch gekennzeichnet, daß der Metallstrang als Litze (22;22a) aus verseilten rostfreien Stahldrähten gebildet ist.

## Claims

**1.** A device for verifying the position of a catheter in the central venous region by ECG-lead, consisting of a hose-shaped catheter (11; 11a) with a catheter hub (12) and of a stylet (21) set into the lumen of said catheter (11; 11a) and being formed of a metal string encoated with plastic, the tip of said metal string being withdrawn at a distance behind the axial opening (30; 30a) of the tip of said catheter (11; 11a) and said distal end of said metal string bearing an electric contact member (26) for connection to an ECG apparatus,

characterized in that

said tip of said metal string of said stylet (21) ends flush with the edge of its plastic sheath (23).

**2.** A device for verifying the position of a catheter in the central venous region by ECG-lead, consisting of a hose-shaped catheter (11; 11a; 11b) with a catheter hub (12) and of a stylet (21a) set into the lumen of said catheter (11; 11a; 11b) and being formed of a metal string encoated with plastic, the tip of said metal string being withdrawn at a distance behind the axial opening (30; 30a) of the tip of said catheter (11; 11a; 11b) and said distal end of said metal string bearing an electric contact member (26) for connection to an ECG apparatus,

characterized in that

a portion (22b) of said tip of said metal string of said stylet (21a) projects beyond the edge of its plastic sheath (23a).

**3.** The device of claim 1 or 2, characterized in that the wall of said catheter (11a; 11b) has holes in its portion (32; 36) free of said stylet.

**4.** The device of claim 3, characterized in that a plurality of small holes (33; 38) are distributed circumferentially.

**5.** The device of claim 1 or 2, characterized in that the distal end of said metal string projects beyond the end of said catheter (11; 11a; 11b) and is firmly connected to a cap-shaped stylet hub (24) adapted for releasable coupling to said catheter hub (12) and bearing said contact member (26).

**6.** The device of one of claims 1-5, characterized in that said metal string is formed as a strand (22; 22a) of twisted wires of stainless steel.

## Revendications

**1.** Dispositif de vérification de la position d'un cathéter placé dans le système veineux central, au moyen de l'établissement d'un électrocardiogramme, constitué par un cathéter en forme de tuyau (11;11a) comportant un embout (12) et un mandrin (21), qui est inséré dans l'ouverture du cathéter (11;11a) et formé par une bande métallique gainée d'une matière plastique, et dans lequel la pointe de la bande métallique est disposée en retrait à une certaine distance en arrière de l'ouverture axiale (30;30a) de la pointe du cathéter (11;11a), et l'extrémité, éloignée du patient, de la bande métallique porte un élément de contact électrique (26) permettant le raccordement à un électrocardiographe, caractérisé en ce que la pointe de la bande métallique du mandrin (21) se termine de niveau avec le bord de sa gaine en matière plastique (23).

**2.** Dispositif de vérification de la position d'un cathéter placé dans le système veineux central, au moyen de l'établissement d'un électrocardiogramme, constitué par un cathéter en forme de tuyau (11;11a;11b) comportant un embout (12) et un mandrin (21), qui est inséré dans l'ouverture du cathéter (11;11a;11b) et formé par une bande métallique gainée d'une matière plastique, et dans lequel la pointe de la bande métallique est disposée en retrait à une certaine distance en arrière de l'ouverture axiale (30;30a) de la pointe du cathéter (11;11a;11b), et l'extrémité, éloignée du patient, de la bande métallique porte un élément de contact électrique (26) permettant le raccordement à un électrocardiographe, caractérisé en ce que la pointe de la bande métallique du mandrin (21a) fait saillie, par une section (22b), par rapport au bord de sa gaine en matière plastique (23a).

3. Dispositif selon la revendication 1 ou 2, caractérisé en ce que la paroi du cathéter (11a;11b) est perforée dans la zone, qui est dégagée du mandrin, de sa pointe (32;36).

4. Dispositif selon la revendication 3, caractérisé en ce que plusieurs petits trous (33;38) sont disposés en étant répartis sur la périphérie.

5. Dispositif selon la revendication 1 ou 2, caractérisé en ce que l'extrémité, éloignée du patient, de la bande métallique, fait saillie sur l'extrémité du cathéter (11;11a;11b) et est raccordée fermement à un embout (24) du mandrin, en forme de capuchon, qui peut être accouplé de façon amovible à l'embout (12) du cathéter et porte l'élément de contact (26).

6. Dispositif selon l'une des revendications 1-5, caractérisé en ce que la bande métallique est réalisée sous la forme d'une tresse (22;22a) formée par des fils d'acier inoxydable torsadés.

FIG.1

EP 0 210 340 B1

# FIG.2

# FIG.3

# FIG.4

FIG.5

FIG.6

FIG.7

FIG.8

FIG.9